# EUROPEAN PATENT APPLICATION

(11) **EP 1 162 275 A1**
(43) Date of publication of application: **12.12.2001**
(21) Application number: 00907410.5
(22) Date of filing: 25.02.2000
(51) Int. Cl.: C12Q 1/68, C12N 15/10

(54) **METHOD FOR LARGE SCALE cDNA CLONING AND SEQUENCING BY CIRCULATING SUBTRACTION**

(30) Priority: 26.02.1999 CN 99102450
(71) Applicant: Shanghai Biorigin Gene Development Co., Ltd, Shanghai 200001 (CN)
(72) Inventor: Mao, Yumin, Shanghai 200433 (CN); Xie, Yi, Shanghai 200433 (CN)
(74) Representative: Viering, Jentschura & Partner
(86) International application number: CN0000036
(87) International publication number: WO0052200

(57) **Abstract**

The present invention provides a method of cDNA sequencing by circulating subtraction, which comprises the steps of: (1) constructing the cDNA original libraries with high quality using the organism tissues as materials; (2) homogenizing the cDNA original libraries of step (1) according to the graded C₀t value, thereby obtaining the homogenized cDNA libraries corresponding to the graded C₀t value; (3) selecting and sequencing the clones from each of the homogenized cDNA libraries of the preceding step; (4) hybridizing and subtracting the homogenized cDNA libraries of the preceding step by using the sequenced cDNA clones; (5) repeating steps (3) and (4) 1-5,000 times. Further, a step of preliminary subtractive hybridization may be included in the method. By using said method, one can sequence cDNA of certain tissues or species efficiently and comprehensively.

## Description

### Field of invention

The present invention relates to the field of genetics, molecule biology and cDNA cloning. In particular, the present invention refers to a method of large-scale and comprehensive cDNA sequencing in any types of cells in one species.

### Background

With the development of biology, especially molecule biology, it is already well known that it is the genetic materials―genes that decide the biological characters. In order to understand all kinds of biochemical process deeply, to discover the relationships between diseases and genes, and to develop a crop with high quality, it is necessary for people to know the DNA information of an organism as completely as possible.

In any species, especially in the eukaryote, the gene expressions have obvious differences from space to space and from time to time. Even for the same organism, the different types of cells will express different genes, and even the same cell in different period may express different genes. For example, the total genes in the human genome are estimated to be 50,000-100,000. (Bishop,J.O., J.G.Morton, M.Rosbash,and M.Richardson.(1974) Three abundance classes in Hela cell messenger RNA. Nature 250:199-204) In any types of cells, only about 10,000 genes can be expressed and the average expression quantity of these genes in a single cell ranges from 200,000 copies to less than one copy. So, a great obstacle exists in the art to sequence as many as possible, not to say the all expressed genes.

With the improvement of the PCR technology, it has been possible to prepare the cDNA library from a single cell. (Li,H., Gyllensten, U.B., Cui,X., Saiki, R.K., Erlich,H.A. & Arnheim, N.(1988) Nature (London) 335,414-417). Further, theoretically, it is possible to prepare a series of cDNA libraries that represent most or even all of the genes. Firstly preparing an cDNA library, then randomly selecting cDNA clones from the cDNA library and performing the large-scale and non-repeated sequencing, has been proved to be an efficient approach to discover new genes.( Adams,M.D., J.M.Kelley, J.D.Gocayne, M.Dubnick, M.H. Polemeropoulos, H.Xiao, C.R. Kerlavage, W.R.McCombie, and J.Craig Venter. (1991)Complementary DNA sequencing: Expressed sequence tags and Human Genome Project. Science 252:1651-1656.Adams, M.D., A.R.Kerlavage, C.Fields, and J.C.Venter.(1993a) 3,400 new expressed sequence tags identify diversity of transcripts in human brain. Nature Genet. 4:256-257.Adams,M.D., M.B.Soares, A.R.Kerlavage,C.Fields, and J.C. Venter. (1993b) Rapid cDNA sequencing (expressed sequence tags) from a directionally cloned human infant brain cDNA library. Nature Genet. 4:373-380.;Adams,M.D., A.R.Kerlavage, R.D. Fleischmann, R.A. Fuldner, C.J.Bult, N.H.Lee, E.F.Kirkness, K.C.Weinstock, J.D.Gocayne, O.White, et al.(1995) Initial assessment of human gene diversity and expression patterns based upon 83 millions nucleotides of cDNA sequence. Nature 377:3-174.; Khan,A.S., A.S.Wilcox, M.H.Polymeropoulos, J.A.Hopkins, T.J.Stevens, M.Robinson, A.K.Orpana,and J.M.Sikela.(1992) Single pass sequencing and physical and genetic mapping of human brain cDNAs. Nature Genet. 2:180-185.; McCombie, W.R., M.D.Adams, J.M.Kelley, M.G.FitzGerald, T.R.Utterback, M.Khan, M.Dubnick, A.R.Kerlavage, J.C. Venter, and C.Fields.(1992)Caenorhabditis elegans expressed sequence tags identify gene families and potential disease gene homologues. Nature Genet. 1:124-131. ; Okubo,K., N.Hori.R.Matoba, T.Niiyama, A.Fukushima, Y.Kojima,and K.Matsubara.(1992) Large Scale cDNA sequencing for analysis of quantitative and qualitative aspects of gene expression. Nature Genet.2:173-179.) The improvement of the technology of large-scale random cDNA sequencing will accelerate the ongoing projects for finding out new genes of human and other species.

However, various technological difficulties in large-scale random cDNA sequencing must be overcome:
First, because the abundance varies from cell to cell and gene expression varies from gene to gene in each type of cell, in order to sequence all the cDNA of one species, it is necessary to obtain cDNA from cells in different growing periods, thus making an intact cDNA library become extremely large. In the cDNA library whose capacity reaches 10⁷ clones, the mRNA from any tissues and even the rare mRNA may be repeatedly cloned, thus making the subtraction and the selection of the lowest genes become almost infeasible. To overcome these difficulties, one should first find out a desirable method to construct a cDNA library, because the ordinary cDNA library usually contains some highly abundant but unwanted clones (referred as "garbage" clones). (Adams,M.D., J.M.Kelley, J.D.Gocayne, M.Dubnick, M.H. Polemeropoulos, H.Xiao, C.R. Kerlavage, W.R.McCombie, and J.Craig Venter.(1991) Complementary DNA sequencing: Expressed sequence tags and Human Genome Project. Science 252:1651-1656.Adams, M.D.,M.Dubnick, A.R.Kerlavage, R.Moreno, J.M.Kelley, T.R.Utterback, J.W. Nagle, C. Fields, and J.Craig Venter. (1992) Sequence identification of 2,375 human brain genes. Nature 355: 632-634.) These "garbage" clones include:
   (1). clones merely having the mRNA poly A trails;
   (2). clones only having the very short inserted cDNA fragments;
   (3). clones only having the 3' end of linker―(dT)₁₈ primer (the primer used to form the first cDNA chain linked to the synthetic linker);
   (4). fused-clones which are produced from the linkage of some different mRNA in the linking reaction and are usually the false cDNA clones.
      The existence of these clones not only seriously hampers the efficiency of the large-scale sequencing, but also destroys the integrity of the sequencing results to a large extent.
      In addition, as a universal rule, the abundance of a cDNA clone in the cDNA library is always consistent with the abundance of the corresponding mRNA in the cell. The analysis of the annealing dynamics shows that, based on the abundance, mRNA in any typical body cells can be classified into the following three classes:
      <1> high abundance mRNAs (These mRNAs are usually of only 10-15 types having highly repeated regions. The amount of these mRNAs is about 10-20% of the total mRNA.)
      <2> moderate abundance mRNAs (These mRNAs are usually of 1000-2000 types having moderately repeated regions. The amount of these mRNAs is about 40-45% of the total mRNA.)
      <3> low abundance mRNAs, i.e., complicated mRNAs (These mRNAs with low copy number usually are of about 15,000-20,000 types. The amount of these mRNAs is about 40-45% of the total mRNA.) (Bishop,J.O., J.G.Morton, M.Rosbash,and M.Richardson.(1974) Three abundance classes in Hela cell messenger RNA. Nature 250:199-204.; Davidson, E.H.and R.J.Britten.(1979) Regulation of gene expression : Possible role of repetitives sequences. Science 204:1052-1059.)
Therefore, if one takes a new clone out of the library after the majority of mRNAs of Classes <1> and <2> have been identified, the possibility that said clone is from one of the sequenced sequences is over 60%. Thus, it is inevitable to carry out plenty of duplicate sequencing to find out a new gene. The great difference of mRNA abundance results in plenty of duplicate and redundant sequencing. Even many of the high quality cDNA libraries commercially available are not the ideal material for large-scale cDNA sequencing.

In order to overcome the above difficulties, construction of high quality cDNA libraries, and homogenization and subtraction of said libraries are advanced and promisingly technologies.

One of the homogenizing methods is the saturation hybridization of DNA genome. **(Weissman,S.M.(1978) Mol.Biol.Med. 4,133-143.)** Although this method makes cDNA library homogenized, but it is unfeasible in practice because it is difficult to provide saturation quantity of rare cDNA to participate in the hybridization. Another method is to utilize annealing dynamics. According to conditions of the second dynamics, the cDNAs are annealed. Because the rare cDNAs anneal much slowly than the high abundance cDNAs, the single-chain cDNA fragments after annealing will be gradually homogenized during the reaction. (That is, the lower abundant cDNA is more likely to be retained, thereby causing the number of the clones with different abundance closer) (Weissman,S.M.(1987) Mol.Biol Med. 4,133-143.; Ko, M. S.H..(1990) Nucleic Acids Res.18, 5705-5711.; Patanjali, S.R., Parimoo,S. &Weissman,S.M.(1991) Proc. Natl.Acad.Sci. USA 88.1943-1947.)The method of homogenization maintains the emergence frequency of every clone in the cDNA library within a small range. According to the practical experience of some scholars, it is impossible to obtain a cDNA library having an identical mole of various cDNA.

The homogenization has the following advantages: (1) Homogenized cDNA library can accelerate the mapping of the clones so as to obtain the disease-associated genes, to improve the efficiency of the subtractive hybridization, to expedite the research on the arrangement of the expressed sequence tag or EST on the chromosomes and their locations on the cloned large DNA fragments from the genome (i.e., extron mapping). (2) By reducing the number of the identical clones, homogenization makes it possible to overlay the cDNA library onto a film in high density. (It is impossible to overlay 10⁷ clones onto a film for unhomogenized library) (3) Using random cDNA sequencing, homogenization can greatly accelerate the development of EST database by increasing the emergence frequency of rare clones and decreasing the proportion of high abundance cDNA.

Selecting the homogenized library, i.e., the library in which the abundances of all clones are essentially same, is beneficial to large-scale sequencing. (Soares, M.B.1994.Construction of directionally cloned cDNA libraries in phagemid vectors. In Automated DNA sequencing and analysis(ed. M.D.Adams, C.Fields and J.C.Venter),pp.110-114. Academic Press, New York,NY., Soares, M.B., M.F.Bonaldo,P.Jelenc, L.Lawton,and A.Efstratiadis. 1994.Consruction and characterization of a normalized cDNA library. Proc.Natl.Acad.Sci. 91:9228-9232.)This has been proved by the experiment finished by Berry etc. (Berry,R., T.J. Stevens, N.A.Walter, A.S.Wilcox, T.Rubano, J.A.Hopkins, J.Weber, R.Goold, M.B.Soares, and J.M.Sikela.1995. Gene-based sequence-tagged-sites(STSs)as the basis for a human gene map. Nature Genet.10:415-423.; Houlgatte,R., R.Mariage-Samson, S.Duprat, A.Tessier, S.Bentolila, B.Lamy, and C. Auffray.1995. The Genexpress Index: A resource for gene discovery and genic map of human genome. Genome Res. 5:272-304.) Calculation Results show that when the C₀t is 5.5, wherein Cₒ represents total DNA concentration whose unit count as nucleotide for mol per liter; t represents time whose unit is second, the content of the high abundance mRNA of the three kinds of mRNA is sharply decreased and the abundances of various clones are restricted in a certain range. (Soares, M.B.1994.Construction of directionally cloned cDNA libraries in phagemid vectors. In Automated DNA sequencing and analysis(ed. M.D.Adams, C.Fields and J.C.Venter), pp.110-114. Academic Press, New York,NY.)

Because a relatively large portion of the human genes have been identified, it is impossible to completely rely on the method of library homogenization so as to avoid duplicate sequencing those genes expressed in several tissues. Therefore, Soares deems that it will bring more advantage in the full-scale sequencing by using the subtractive cDNA libraries that enrich the low expressed genes and the unidentified genes.

In the attempts to improve the representation of the longest cDNA clones in the library, Soares et al. established four methods for constructing the homogenized library and successfully constructed various homogenized libraries including fifteen human libraries, three mice libraries, two rat libraries and one *Schistosoma mansoni* library. The human libraries and the mice libraries were provided to the IMAGE ( an organization for molecular analysis of human genome and expressing). (Lennon,G.G., C.Auffray, M.Polymeropoulos, and M.B.Soares.1996. The I.M.A.G.E. Consortium: An integrated molecular analysis of genomes and their expression. Genomics 33:151-152.). A total of 315,408 expressed sequence tags (ESTs) were obtained from these libraries. The above reference gives a detailed description and a comparison of these four methods for homogenizing cDNA libraries. Furthermore, a simple scheme for constructing the subtractive cDNA library is also described in details in this reference.

Although there are some methods of homogenization and subtraction, there are still many disadvantages. In the published articles of Soares et al. in 1994, one method of homogenization, referred hereinafter as "Soares Method 1", was described in details. (Soares, M.B.1994.Construction of directionally cloned cDNA libraries in phagemid vectors. In Automated DNA sequencing and analysis(ed. M.D.Adams, C.Fields and J.C.Venter),pp.110-114. Academic Press, New York,NY.) Said method was used to construct the 1NIB and 1NFLS homogenized libraries, from which 45,192 and 86,088 ESTs were obtained, respectively. Using the method of homogenization, the abundance of clones can be limited into a narrow range. However, the comprehensive identification of these two libraries by Southern analysis shown that the truncated clones are more easily reserved than the corresponding intact clones during the homogenization process of Soares Method 1.

Why are the truncated clones more easily reserved than the corresponding untruncated clones during the homogenization process of the Soares Method 1? (Soares, M.B.1994.Construction of directionally cloned cDNA libraries in phagemid vectors. In Automated DNA sequencing and analysis(ed. M.D.Adams, C.Fields and J.C.Venter),pp. 110-114. Academic Press, New York,NY.). In order to answer this question, first take a look at Soares Method 1 which mainly comprises the following steps.
(1) annealing the single-stranded DNA (ssDNA) prepared from a cloned cDNA library with the oligo(dT)₁₈ primers;
(2) in the presence of deoxy- and dideoxy- NTPs, performing the primer extending reaction to produce the 3' extension products of the non-coding chain with a length of 200-300bp;
(3) purifying a portion of the double-stranded circular DNA produced in the above step by HAP chromatography;
(4) denaturing the double-stranded circular DNA obtained in Step (3), and renaturing them in a relatively low C₀t (C₀t is about 5-10);
(5) purifying the remaining single-stranded circular DNA by HAP to obtain the homogenized library;
(6). converting the single-stranded circular DNA into the double-stranded circular DNA;
(7). introducing the double-stranded plasmid into the host cell by electoporation.

Because the probability for producing the truncated cDNA is lower than that for producing the corresponding untruncated cDNA, in the reaction, the cDNA amplification products is easier to anneal with an intact cDNA having an overlapping region than the template itself (i.e., the truncated cDNA). On the other hand, the amplification products of the untruncated cDNA are most likely to be annealed with their templates, and not with the untruncated cDNA, because the amount of the untruncated cDNA is larger than the truncated cDNA and the possibility that an overlapping region exists between the extension fragments produced by the untruncated clones and the truncated clones is very low. The result is that the untruncated single-stranded clones may renature with more than one extension products which are not overlapped with each other, while the truncated clones remain single-stranded so as to be retained in the elution solution after purifying by HAP, thereby forming a port of the homogenized library

In order to solve these problems, Soares designed Methods 2-1 and 2-2, whose processes are almost the same except the hybridization conditions. Using these methods, one can use the plasmid DNA prepared from the original library as a template to synthesize RNA. Said RNA fragments are then used as the drivers to hybridize with the same library of single-stranded circular DNA. These two improved method increase the expressivity of the longest cDNA clones in the homogenized library.

However, in the libraries constructed by Methods 2-1 and 2-2, Soares found some cDNA clones, e.g., the alpha- lactoprotein gene in the 5Nb2HFLS20W liver/spleen library and the G3PD gene in the breast library, which are much more difficult than any other clones to be homogenized. Although this problem was somewhat solved in Method 2-3, the degree of homogenization thereof can not reach the same level of homogenization of Method 1. Further, Method 2-3 is not a real homogenization process, because the purpose of this method is not to make the abundances of all the cDNA clones to be the same or similar but to reduce the expressivity of clones with the highest abundance.

Soares, based on the combination of Methods 1 and 2, attempted to establish Methods 3 and 4 so as to achieve not only the sufficient homogenization as in Soares Method 1, but also the high expressivity of the longest cDNA clones as in Methods 2-1, 2-2 and 2-3. However, this is only a partial improvement and the effect is not significant.

So it is an urgent need in the art to develop a technical solution for efficient, complete and large-scale sequencing of cDNA so as to powerfully accelerate the development of molecular biology, genetics, medication and breeding, etc.

The purpose of this invention is to provide a method suitable for sequencing cDNA effectively and extensively. This method is suitable for large-scale sequencing of cDNA in various eukaryotes, especial the higher plants and animals, e.g., the mammals.

### Summary of Invention

The purpose of this invention can be achieved by the following technical solution. A method of sequencing cDNA which comprises the steps of:
(1) constructing cDNA original libraries in which the length of the inserted fragments is 0.5-3.0 kb, by using tissues of organisms as materials;
(2) homogenizing the cDNA original libraries of step (1) according to the graded C₀t value, thereby obtaining the homogenized cDNA libraries corresponding to the graded C₀t, wherein C₀ is concentration of the total DNA and the unit thereof is mol/L based on the number of nucleic acids; and t is the renaturing time and the unit thereof is second;
(3) selecting and sequencing 5-500 clones from each of the homogenized cDNA libraries of the preceding step;
(4) synthesizing probes corresponding to the sequenced sequences by using the sequenced cDNA clones, and hybridizing and subtracting the homogenized cDNA libraries of the preceding step with said probes, thereby removing the sequenced cDNA clones from the cDNA libraries and forming the subtracted homogenized cDNA libraries;
(5) repeating Steps (3) and (4) 1-5,000 times.

### Detailed description of the Invention

Though study, the inventors has discovered a method to efficiently and completely sequence the genes expressed in an organism, a tissue, or a cell. This method includes the following steps: constructing a high-quality cDNA original library to ensure that the clones in the library correspond to all of or almost all of mRNA; dividing the C₀t value into different grade so that the cDNA of different abundance can be reserved in one or more homogenized cDNA libraries; removing the sequenced cDNA clone from the homogenized cDNA libraries by hybridization and subtraction. Therefore, after hybridization and subtraction, the homogenized cDNA libraries only contain the unsequenced cDNA clones, thereby significantly improving the efficiency of the sequencing in the next cycle.

In order to study the gene expression in different tissues, the method of the invention may comprise: using different tissues of the organism as materials to construct cDNA original libraries of different tissues; homogenizing said cDNA original libraries respectively to obtain homogenized libraries of different tissues; hybridizing and subtracting among said homogenized cDNA libraries of different tissues, thereby forming the homogenized cDNA libraries subtracted by hybridization. Further, hybridization and subtraction between cDNA original libraries of different tissues can be done so as to obtain a cDNA original library which contains differentially expressed cDNA.

In this invention, "different tissue"means any different tissues due to different organisms, different types, different phases of development and/or different status of pathology. The examples of different tissues include, but are not be limited to, the tissues of different types in an organism, such as human brain and muscle; the tissues of different phase of development in an organism, such as brain in the embryonic and adult stage; normal tissues and pathologic tissues in an organism, such as a normal brain and a brain of senile dementia; and tissues of different organisms, such as the brain of rats and rabbits, etc.

For the constructed cDNA library to be sequenced, one may first hybridize and subtract it with a cDNA original library of different tissues or a homogenized cDNA library so as to sequence the cDNA expressed differentially. In particular, a cDNA library of pathologic tissues can be hybridized and subtracted with a cDNA library of normal tissue so as to obtain the cDNA expressed differentially in the pathologic tissue and sequence it. Similarly, the cDNA clones containing genes expressed differentially in the different phases of development can be obtained by hybridizing and subtracting between the cDNA libraries of different phases of development of the same tissue.

Furthermore, in order to improve the efficiency of sequencing and reduce duplicate sequencing, one may synthesize artificial probes in the steps of hybridizing and subtracting based on the information of the known cDNA sequences, especially for the organism, e.g., human, whose cDNA sequences are relatively well known, or based on other sequenced clone. Said probes are then used to hybridize and subtract the cDNA original library or the homogenized cDNA library.

The sequencing method used in the invention can be any of the conventional method in the art, manually or automatically. A useful procedure of DNA sequencing comprises the steps of:
for the sequenced fragments, determining whether they are new cDNA clones;
for the new clones, analyzing the integrity of 5' end;
for the new clones having the integral 5' end, sequencing continuously until obtaining the full-length sequences.

In the invention, the C₀t value is typically divided into 3-8 grades in the process of homogenization. However, it is also possible to divide it into more grades, such as 10 grades or less grades, e.g., 2 grades. Preferably, C₀t is divided into 3-5 grades.

In one embodiment, the C₀t is divided into 3 grades as follows: 0 < C₀t ≼1, C₀t =1-50, and C₀t ≽ 50, thereby, from the same cDNA original library, obtaining the homogenized libraries selecting from the group consisting of (a) highly repeated homogenized cDNA libraries, (b) moderate repeated homogenized cDNA libraries, and (c) lowly repeated homogenized cDNA libraries. In another embodiment, the C₀t is divided into 4 grades as follows: 0<C₀t≼0.5; C₀t=0.5-10; C₀t=10-50 and C₀t≽5. Especially for sequencing the low abundance cDNA, the C₀t is preferred to be divided into more grades in the range of 0-5.

One key for successful sequencing is to obtain a high quality cDNA original library that contains most or all of cDNA, and less "garbage" clone. Therefore, an appropriate amplification technique of high efficiency should be used to amplify the extracted mRNA to form the corresponding cDNA. The examples of the technique include, but are not be limit to, mixed reverse transcriptase technique, SMART PCR technique, nucleotide capping technique and the combination thereof. Furthermore, it is necessary to separate the amplified fragments and remove the short fragments (e.g., less than 200bp). Then, the cDNA original library can be constructed by inserting the fragments whose lengths meet the requirement into the appropriate vector.

A method useful for constructing the cDNA original library in the invention comprises the following steps:
extracting mRNA, amplifying mRNA to obtain the corresponding cDNA by a technique selected from the group consisting of : mixed reverse transcriptase technique, SMART PCR technique, nucleotide capping technique and the combination thereof;
separating and collecting cDNA fragments of 0.5-3.0kb;
cloning the separated cDNA fragments into proper vectors;
separating the vectors comprising the inserted fragments of 0.5-3.0kb;
transforming into proper bacteria, thereby obtaining the cDNA original libraries.

Further, the separation and collection of cDNA fragments of 0.5-3.0kb can be carried out by electrophoresis and cutting gel, or by gel chromatography purification; and the separation of the vectors comprising the inserted fragments of 0.5-3.0kb can be carried out by reversed phase HPLC.

Another important key to this invention is that a series of technical steps for constructing high quality cDNA original library are first proposed by the inventors. The purpose of these steps is to reduce the number of garbage clones included into the cDNA original library from the very beginning and to the largest extent. The most intact cDNA is amplified by the highly efficient nucleic acid amplification techniques, and the clones are purified prior to or right after cloning into the vectors. These technical steps ensure the cDNA in the cDNA original library is long enough.

Furthermore, Scoares, et al (Proc.Natl.Acad.Sci.USA Vol.91, pp.9228-9232) reported that about 13% clones in IB library (from human mRNA) lack the poly (A) tail. It is proposed that abnormal priming during the amplification may cause this. Therefore, in order to further improve the quality of cDNA original library, a treatment with a d(T) 18-25 affinity column can be used to greatly decrease the amount of the clones that lack the poly (A) tail, thereby constructing a better cDNA original library.

In order to determine various genes in an organism and ensure a smooth and efficient large-scale random cDNA sequencing, one desirable method is to construct enough or all of the high quality cDNA original libraries from one species (such as human). For example, these cDNA original libraries can be from human, mouse, rats or schistosome. These cDNA original libraries can also be from the normal tissues or typical pathological tissues of an organism, or from the tissues in the various development phase. The materials used to construct the cDNA original library are various tissues. The examples include, but are not be limited to, (a) brain of human infant, liver and spleen of human embryo, human parturient placenta, human placenta aged 8-9 weeks, human breast, adult brain, human retina, human pineal gland, human ovary tumor, human melanin cell, the heart of human embryo, human parathyroid gland tumor, human senile fibroblast, human sclerosis focus, lung of human embryo, (b) 19.5 day old mouse embryo after amphimixis, 17.5 day old mouse embryo after amphimixis, 13.5-14.5 day old mouse embryo after amphimixis, (c) heart and spleen of rat, (d) adult schistosome of 8 week and so on.

The reason why it is necessary to obtain enough high quality cDNA original libraries is because, in a single cell, about 1/3 of the expressed mRNAs have 1-10 copies (Galau, G.A., Britten, R.J.&Davidson, E.H.(1977) Proc. Natl.Acad.Sci.USA 74,1020-1023.). A single cell can express 10,000 mRNA or more. If each mRNA produces 2 or 3 cDNA in a length of 500-2000bp, then the expressivity of each cDNA is 1/30,000 after homogenization. The expressivity of individual cDNA is lower if the cDNA is from complex tissues. The expressivity of each fragment is lower than 1/100,000 if the library originates from all of the mRNA expressed in different phases.

Therefore, the fully representative library, in which the expressed sequences of various cells are included, can be obtained by the following steps: the libraries are constructed from a proper amount of organs or tissues, pre-homogenized; and then each library is homogenized specially.

Secondly, another important key in this invention is that we proposed to homogenize the high quality library under enough and different C₀t, based on sufficient high quality cDNA original libraries. So, all of the cDNA from a normal tissue or typical pathologic tissue can be distributed in the various homogenized sublibraries. Each sublibrary represents a set of cDNA whose homogenization is higher and more specific. The total of these sublibraries prepared from a cDNA original library of a normal or pathologic tissue correspond to the total cDNA of the normal or typical pathologic tissue.

Furthermore, manifold homogenized sublibraries, which only correspond to the normal tissue or typical pathologic tissue, can be obtained by subtraction. The subtraction includes the step of using the probes, which are prepared from the sequenced cDNA clones, to hybridize with the cDNA original library or sublibraries to remove the sequenced cDNA clone.

In the art, the artisans usually try to find several optimal C₀t so as to solve the question and fails. They deem hybridization as a simple secondary dynamics reaction, and anticipate that the expressivity of the most abundant component in single-stranded DNA is less than twice the least abundant component, if 50% of the least abundant component are renatured in the mixture. In fact, hybridization is much more complex than anticipated. Hybridization of the most abundant component is faster than anticipated. In the early studies, expressivity of the most abundant component in single-stranded DNA was even lower than that of the least abundant component. The main reason for this effect is that double-stranded fragments probably continue to hybridize with the remaining single-stranded cDNA. So the solution in the art is to select appropriate timing of renaturation and concentration of the renaturation reactant so that the effect can be reduced to an acceptable level. Furthermore, adding hydroxyapatite in the various phase of reaction to remove the double-stranded products can further reduce the effect. But hybridization and subtraction with only a few of Cₒt values can not fully solve the problem and the results are not satisfied.

Moreover, in Smith,M.J.,Brititen,R.J.&Davidson,E.H.(1975)Proc.Natl.Acad.Sci. USA72, 4805-48 09.; Marrow, J.F.(1974) Doctoral thesis (Stanford Univ., Standford, CA), PP,101-190.Mol.Cell.Biol.10,243-253, the experimental results did not conform with the prediction based on the secondary dynamics law. First, the most abundant cDNA decreases more quickly than predicted, and the number thereof in the single-strand mixture is less than that of some low abundance cDNAs. Second, even if during the shortest renaturation time, considerable rare cDNAs are found in the double-stranded mixture (Both are inconsistent with the prediction). The explanation for the first abnormal phenomenon is described in this literature (Smith,M.J., Britien, R.J.&Davidson,E.H. (1975) Proc.Natal.Acad.Sci.USA 72,4805-4809.; Marrow, J.F. (1974)Doctoral thesis (Stanford Unive.,Stanford,CA), PP, 101-190. Mol.Cell.Biol.10,243-253.). During the process of renaturation, if there is a homologous region, long fragments and short fragments from the same kind of mRNA will hybridize and produce a single-stranded tail. Thus, this structure can pair with the homology fragment in the remaining single-stranded DNA and does not follow the secondary dynamics reaction law. This dynamics action causes the additional convert from single strand to double strand. So, even if the very low abundance mRNAs, such as c-myc and TCR, may exist in the mixture of single-stranded components and the mixture of double-stranded components during any renaturation time.

Soares, et al (Proc.Natl.Acad.Sci.USA Vol.91,pp.9228-9232) constructed two human fetal brain libraries (¹NIB library and ²NIB library) under two C₀t values of 5.5 and 2.5. It is found that the frequency of occurrence of some clones decrease in the ¹NIB library but increase in the ²NIB.

For example, based on the calculation of estimated mRNA abundance in brain, the optimal degree of enrichment of mRNA is achieved when C₀t is 5-10. However, this is only suitable for relatively abundant cDNAs. If C₀t is too low (≼1), only the most abundant mRNAs (Class <1>) are enriched; moderate abundance mRNAs ( Class <2>)are unable to be enriched. On the other hand, if C₀t is too high(≽50), the degree of enrichment of the most abundant mRNA is insignificant while expressivity of the most complicated mRNA ( Class <3>) becomes high. So, it is proposed in the invention that, for a cDNA original library, all of cDNAs can not be distributed into the different homogenized libraries unless different C₀t values are selected to construct a series of homogenized libraries, depending on the different organism sources and/or sequencing purposes.

As the third important key of the invention is the circulating subtraction of the homogenized library after every selected clone from the homogenized library is sequenced. Thus, the sequenced clones are removed and the unsequenced clones are retained to be sequenced in the next cycle. Therefore, the sequencing efficiency is improved significantly and the possibility for duplicate sequencing is decreased dramatically.

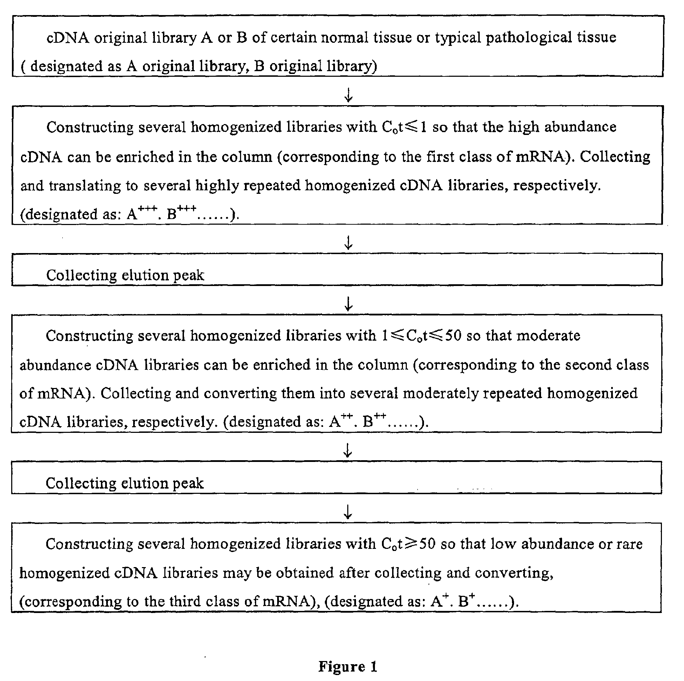

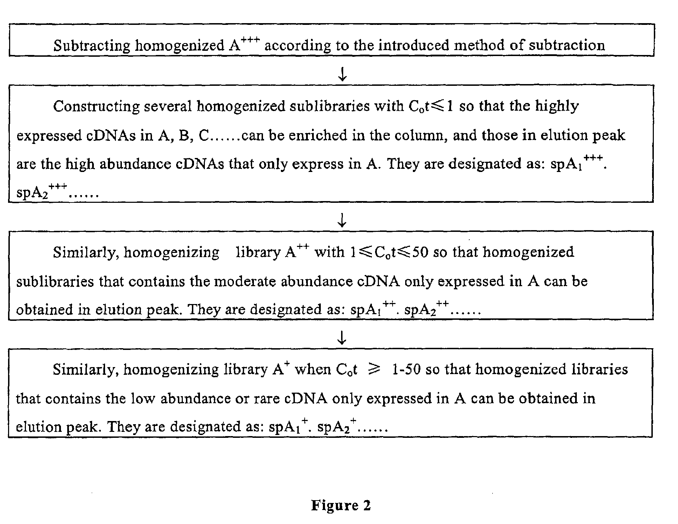

In particular, the method of the invention includes the following steps:
(1) Constructing high quality cDNA original library.
   For the conventional methods of constructing cDNA library, see the above description. Preferably, in this invention, the following one or more of techniques are used: mixed reverse transcriptase technique ( Usage FD heat-resistant reverse transcriptase; Fudan transaction 37(2):225-228; China Biochemistry And Molecular Biochemistry 14(2):170-174,1998); SMART PCR technique (Clontech, SMART™ PCR cDNA Library Construction Kit User Manual (PT3000-1); (Kazuo Maruyama and Sumio Sugano(1994) Oligo-capping: a simple methods to replace the cap structure of eukaryotic mRNAs with oligoribonucleotides. Gene 138, 171-174.Carninci, P. et al. (1996) High-efficiency full-length cDNA cloning by biotinylated CAP trapper. Genomics 37,327-336.). Thus, the ratio of full-length cDNA in the whole products is increased. In order to assure the ratio of full-length cDNA in the whole cDNA original library, it is preferable to isolate the amplified cDNA fragments with the desired length (usually at least 200bp) after amplification, and clone them into appropriate vectors. Finally, the resultant vectors are loaded onto the HPLC reverse column and the peaks containing the vectors that are inserted by fragments (e.g., 0.5, 3.0.2kb) are collected. Said collected vectors are transformed into the appropriate bacteria, thereby obtaining high quality cDNA original library.
   Using the above steps, the ratio of garbage clones in the high quality cDNA original library is so small that it can be neglected.
   One desirable strategy is to get the high quality cDNA original libraries of different normal tissues or typical pathologic tissue of an organism as many as possible so as to obtain the cDNA original library which overlays all of or almost all of expressed genes of the organism.
(2) Homogenizing under the graded C₀t values
   Then, using the methods of homogenization in the art, the high quality cDNA original libraries of the preceding step is homogenized. The difference is to divide C₀t into several grades and to homogenize under different C₀t values. Because there is no best C₀t value under which one can not only achieve the homogenization but also retain all kinds of cDNA clones corresponding to different abundences. On the contrary, only if a series of homogenized libraries are constructed under different C₀t values, can all of cDNAs be distributed into different homogenized libraries.
   In particular, depending on the cDNA original libraries from different origins, one can choose enough different C₀t values, wherein C₀ represents total DNA concentration whose unit is mole of the nucleic acid per liter and t represents time whose unit is second(s).
   For example, the C₀t is divided into three grades such as high, moderate, and low grades, and several different C₀t values are selected in each grade. Therefore, one cDNA original library will be divided into three classes of homogenized libraries, i.e., high abundance homogenized cDNA libraries (C₀t ≽ 1), moderate abundance homogenized cDNA libraries (C₀t=1-50), and low abundance homogenized cDNA libraries (C₀t ≽ 50). Each class of homogenized libraries can be further divided into more homogenized sublibraries. The process is shown in Figure 1.
(3) Pre-subtraction by hybridization
   The process of pre-subtraction by hybridization is optional. In order to find target genes directly or quickly, the homogenized libraries may be pre-subtracted by hybridization. For example, pre-subtraction by hybridization can be performed among the homogenized cDNA libraries of the same abundance (such as high abundance) from different tissues, such as brain and liver, so as to rapidly determine the cDNA expressed differentially. Alternatively, pre-subtraction by hybridization can be performed between the cDNA libraries of normal and pathologic tissues of the same tissue (such as brain tissue). Further, pre-subtraction by hybridization can be performed among the homogenized cDNA libraries of different abundances of the same normal tissue (such as high and low abundance homogenized cDNA libraries) so as to focus on the sequencing of low abundance cDNA.
   Therefore, on the basis of existing homogenized methods, subtractive homogenized libraries may be obtained after pre-subtraction by homogenization among different cDNA libraries.
   The process is shown in Figure 2, wherein non-A cDNA original library (including B original library, C original library, etc.) are used as templates to synthesize DNA or RNA fragments which are used as drivers. The drivers are the single-stranded DNA or RNA fragments hybridizing with the original library.
(4)Selecting clones for sequencing
   According to the conventional sequencing method in the art, 5-500 clones are selected randomly from the homogenized cDNA libraries, which have been treated with or without pre-subtraction by hybridization, of the preceding step, judged whether they are the new cDNA clones. If so, integrity analysis of 5' terminal will be carried out until obtaining full-length sequences.
(5) Subtractive hybridization
   The probes are synthesized on the basis of sequenced cDNA libraries consisting of all the long sequenced cDNA clones, and hybridized with the cDNA original libraries, or the homogenized libraries or the subtracted homogenized libraries obtained from the previous step. Thus, the cDNA clones that have been sequenced in the previous step are removed from the libraries and the unsequenced clones are retained. The efficiency of large-scale sequencing is therefore remarkably increased. The retained unsequenced clones make up the subtractive homogenized cDNA libraries, which are used in the next cycle.
(6) Repeating the above Step (4) of sequencing and step (5) of subtractive hybridization
   By repeating the sequencing and step (5) of subtractive hybridization, new sequences are sequenced efficiently and the unsequenced cDNA reserved for the next cycle. There is no limit for the number of repeating or cycle, usually 1-5000 times. The number depends on the circumstances and the need, for example, on the basis of whether the desired genes have been obtained, or on the amount of clones in each cycle.

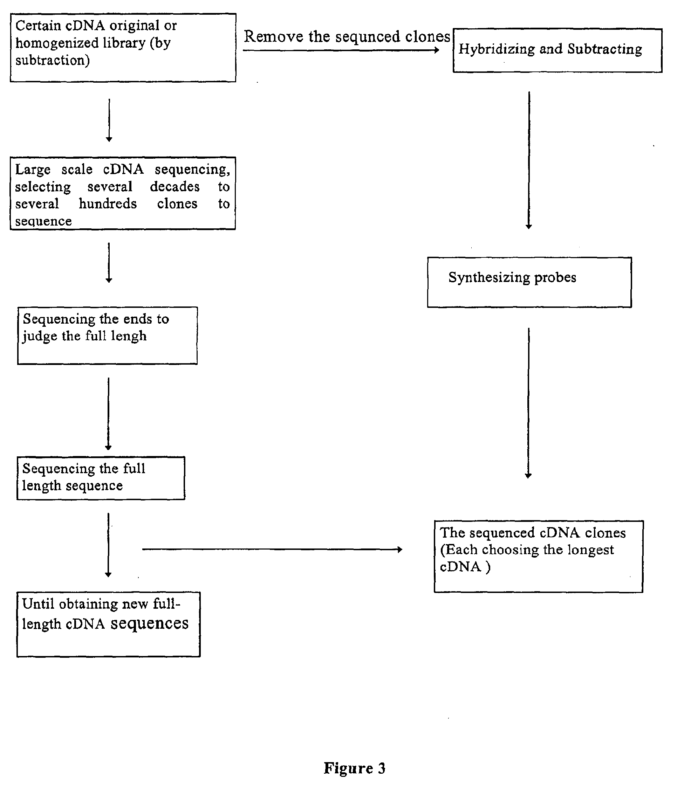

This process is shown in Figure 3.

### Examples

The examples are provide to further elucidate the invention. These examples are only used to explain but not to limit the invention.

### Example:

### General methods

### (A)HPLC reversed phase chromatography

Reversed phase chromatography (RPC) has high resolution. Because the material can tolerate high press, it can be used in HPLC column and make isolation efficiently and rapidly. As a result, it becomes more and more widespread in the isolation and purification of nucleic acid. It also has the following advantages:
(1) Isolating oligonucleotide of different sizes. The resolution is high, especially in isolating copolymerized adenine nucleotide of 10-130bp. It can isolate fragments differing by only one nucleotide. The recovery ratio is high and the purity of the recovered sample is good.
(2) Isolating fragments of DNA restriction endonuclease. It can isolate fragments differing only by 4bp.
(3) Isolating and purifying plasmid DNA molecular of cc type. When isolating the crude products of plasmid DNA on RPC column, the purified cc molecular can be obtained.

Moreover, HPLC column can also be used to isolate and purify plasmid, it can be used to remove the polluting protein and other nucleic acid (such as RNA of high molecular weight), and efficiently isolate plasmids differing by 100-200bp. The common operating conditions are as follow:
Column: Bio-Gel plasmid column
Size: 50 x 7.8mm
Cetolog number: 125-0537
Sample: PUC₁₈ 100ug
Mobile phase:
   A: 0.02M potassium phosphate, 50% formamide, pH6.7
   B: 0.02M potassium phosphate, 50% formamide, pH6.7, 1.5M KCl
      Gradient: 50%B 10min, 50-70%B 30min
      Flow rate: 1.0 ml/min
      Detection: 1uv@ 270nm

### (B)hydroxyapatite column chromatography:

The method of isolating single-stranded DNA and double-stranded DNA with hydroxyapatite has been mentioned in details in the literature (Britten, R. J., Graham, D. E. & Neufeld, B. R. (1974) Methods Enzymol. 29, 363-418.). The concentrations of sodium dihydrogen phosphate used for eluting DNA single-stranded components and double-stranded components were 0.1M and 0.35M, respectively. However, as the single-stranded components of different sizes obtained from M13mp13DNA cleavaged by HaeIII, the double-stranded DNA of less than 200bp would also be eluted by 0.1M phosphate buffer. In order to avoid the pollution to single-stranded DNA by this kind of tiny double-stranded DNA fragments, all the DNA components used in process of homogenization were over 400bp.

The hydroxyapatite (model: DNA-grade Bio-Gel HTP) produced by Bio-Rad Inc. was suspended in 0.01M sodium dihydrogen phosphate (pH7.0) containing 0.1% SDS in the proportion of 1:10. The homogenate was then poured into chromatography column (volume, 1.0ml) containing a water jacket, the flow rate of eluent was 8-10ml/hr. Its binding and eluting characters were tested by HaeIII digestion products of M13mp18 single-stranded DNA and 1kb double-stranded DNA sequence ladder (Life Technologies Inc.) as the marker for double-stranded DNA. The temperature of the chromatography column was maintained at 60 °C by circulative hydrographic basin produced by Neslab Inc. The calibration of the conditions of single-stranded and double-stranded DNA elution was completed by a series of grading gradient sodium dihydrogen phosphate buffer, the concentrations were from 0.05M to 0.4M and increased at an average rate of 0.02M. All the buffers contained 0.1% SDS.

### (C) Desalination and concentration of the elution peak

The single-stranded DNA eluted from the hydroxyapatite column was not suitable for PCR amplification. This is because: (i) sodium dihydrogen phosphate and SDS of high concentration existed, inhibiting the activity of Taq DNA polymerase; and (ii) the concentration of the single-stranded DNA in the eluent was too low. The following methods could be used to solve the problem: filtration with Centricon-30, purification with Glassmilk (according to the handbook provided by manufacturer), concentration with n-butanol and iso-butanol, dialysis after lyophilization, etc. Among these, the method of filtration with Centricon-30 was chosen to recover the driblet single-stranded DNA (Stafford, D. W. & Bieber, D. (1975) Biochem. Biophys. Acta. 378, 18-21). Because the recovery ratio of this method reached 90% and it could remove the salt sufficiently.

The chromatography column eluent was centrifuged and filtrated through Centricon-30 filter membrane so as to concentrate the volume of the eluent from 10-12ml to 100-200ul, then washed with sterilized water and TE buffer (10mM Tris. HCl, pH8.0/1mM EDTA) at least for three times.

### Example 1

### Construction of high quality cDNA original libraries from human normal tissue or typical pathologic tissue and homogenized and subtracted cDNA sublibraries only expressed in healthy infant brain, and the large scale cDNA sequencing.

The materials included in this example for construction of cDNA original libraries were as follows (As sequencing went on or according to requirements, cDNA original libraries from other normal tissue or typical pathologic tissue could be added):
Human infant brain, human embryonic liver and spleen, human full-term placenta, human 8-9 weeks' placenta, human breast, adult brain, human retina, human conarium, human oophoroma, human melanocyte, human embryonic heart, human parathyroid cancer, human senile fibroblast, human poly-sclerotic focus, human embryonic lung.

In details, the materials were:
(1) human infant brain, from a 72-day female infant died of spinal amyotrophy;
(2) human liver and spleen, from a healthy 20-week female embryo;
(3) all the cell poly(A)+mRNA, from healthy breast tissue obtained in breast plastic operation;
(4) all the adult brain RNA, from a 55-year old man died of aortic aneurysm break, brain tissue (including right and left cerebrum, parietal bone, temporal bone, occipital cortex infracortical white matter, basal ganglion, thalamencephalon, mesencephalon, bridge, caudex dorsalls), got 17-18 hours after death;
(5) all the human healthy retina cell RNA, from a 55-year old Caucasian male;
(6) human healthy conarium, from three different races: (i) a 48-year old Caucasian male; (ii) a 18-year old Caucasian female; (iii) a 20-year old Negro American;
(7) all the human oophoroma cell mRNA, from a 36-year old third-stage mastoid cystadenocarcinoma patient, surface diffusion and metastasis having occurred;
(8) all the human melanocyte DNA, from healthy foreskin;
(9) healthy embryonic heart and lung, from samples of a 19-week embryo;
(10) human parathyroid cancer, from accidental adenoma;
(11) cytoplasm mRNA, from human healthy senile fibroblast which was replaced by healthy epidermis fibroblast and whose phenotype was cell body increased, applanation, cleavage stops (add 5- bromouracil. 48 hours later, the infiltration rate was lower than 2%).

During construction, human embryo brain was constructed with Lamda(λ) BA vector, the cDNA libraries of other materials were constructed with pT₇T₃-Pac vector. The cloning site of the embryo liver and spleen cDNA libraries was between enzymatic cleavage sites of PacI and EcoRI, the cloning site of the infant brain cDNA libraries was between enzymatic cleavage sites of NotI and HindIII, the others were all between enzymatic cleavage sites of NotI and EcoRI.

For example, construction of cDNA libraries from healthy female infant brain was as follows:
Poly(A)⁺RNA was extracted from healthy female infant brain (72 days) and used to construct cDNA libraries. Synthesize oligonucleotide 5'-AACTGGAAGAATTCGCGGCCGCAGGAAT₁₈-3' as the primer to synthesize single-stranded cDNA, the oligonucleotide had a NotI site (underlined). Amplifying by nucleotide capping would improve the ratio of full-length cDNA in the products. After electrophoresis, the 2.0kb cDNA fragments were cut and collected from the gel, then linked with the linker of HindIII. After digestion with NotI, the cDNA was linked with phage L-BA that contained HindIII and NotI sites. Vector L-BA derived from pEMBL-9(+) (Dente, L., Cesareni, G. & Cortese, R. (1983) Nucleic Acids Res. 11, 1645-1655.). L-BA contained ampicillin resistance gene, origin of replication of plasmid and f ₁ phage, and multi-cloning site (5' HindIII-BamHI-NotI-EcoRI 3') of. The recombined phage and the helper phage M13K07(Vieira, J. Messing. 1987. Production of single-stranded plasmid DNA. Methods Enzymol. 153: 3-11) were transfected into bacteria, and the double-stranded DNA were converted into annular single-stranded cDNA which comprised the inserted cDNA fragments.

Construction of cDNA libraries of the other materials were the same as the cDNA libraries of healthy female infant brain, except that healthy female infant brain was replaced by other materials; and Lamid BA was replaced by vector pT7T3-Pac and NotI/ HindIII was replaced by the corresponding restriction endonucleases (PacI/EcoRI or NotI/EcoRI).

### Preparation of DNA for single-stranded cDNA original libraries

After purification with reversed phase HPLC and affinity column, the DNA obtained from IB libraries was transformed into DH5α F5' by electroporation. The strain was incubated in an ampicillin-containing medium at 37 °C until its OD₆₀₀ reached 0.2. The helper phage whose content exceeded 20 times was super-infected. 4 hours later, the single-stranded DNA was obtained (Vieira, J. Messing. 1987. Production of single-stranded plasmid DNA. Methods Enzymol. 153: 3-11).

In order to decrease the occurrence of RF DNA, the obtained 20ug DNA was incised with PvuII(PvuII can only incise double-stranded DNA), then extracted with phenol/chloroform, diluted by adding 2ml loading buffer (0.12M sodium phosphate buffer, pH6.8/10mM EDTA/1%SDS), then purified at 60 °C with hydroxyapatite chromatography column, the column was pre-equilibrated with the same buffer (1m1 column bed, 0.4 hydroxyapatite). The column was eluted with 6ml loading buffer, and the solution was combined with fractions passed through the column. The sample was extracted twice with water saturated 2-butanone, once with anhydrous 2-butanone and once with water saturated phenol (3-time extraction solution was added each time). Then the sample was allowed to pass through Nensorb column (DuPont/NEN) to remove the saline ion, precipitated with ethanol, then run on with low melting point agarose gel electrophoresis so as to remove helper phage DNA and tRNA and oligo-ribonucleotide produced by RNase A digestion during purification. The region containing single-stranded libraries DNA on the gel was incised, digested with β-agarase (New English Biolabs). Finally, DNA was purified and precipitated with ethanol.

The above constructed high quality cDNA libraries (IB) from brain and other materials had the following characters: the average length of the inserted fragments in cDNA was 1.7kb; in general, the genetic information within the encoding region could be obtained by sequencing the 5' end; the poly(A) tail of mRNA was short; more useful information could be obtained by sequencing the 3' end; the occurrence of non-recombined clone was very few (0.1%); sequencing more than 2000 clones shown no chimeric cDNA.

### Construction of homogenized cDNA libraries

In common sense, 3' non-encoding region pairing with its transcript is almost unique, so it can be predicted that 3' non-encoding region only pairs with its complementary strand. On the contrary, because the encoding region usually represents members of the oligo-gene or multi-gene family, cross hybridization within the encoding region often results in the decrease of sparse cDNA in libraries during homogenization. The method of the inventors used here can prevent this possibility. At the beginning, the single-stranded annular cDNA was used as a template to synthesize a short complementary strand from 3' terminal and control the length of the strand in a very short range (200±20nt). Because the average length of the mRNA 3' non-encoding region was 750nt (Hawkins, J. D. (1998) Nucleic Acids Res. 16, 9893-9908.), most of the synthesized complementary strand engaged in anneal should lack encoding sequences. However, it was necessary to purify the products by hydroxyapatite chromatography after partial elongation, because when the single-stranded DNA in the IB libraries, which lacked poly(A) tail and could not synthesize primer, passed through the column at the first time, about 0.1% of the DNA would be non-specifically bound onto the column.

The method included the following steps: denaturing, annealing, then purifying the unassociated annular DNA (homogenized libraries) by hydroxyapatite chromatography and transforming into the bacteria by electroporation. In order to synthesize the nucleotide of about 200nt by restricted elongation, 9pmol of oligonucleotide primer 5'-GGCCGCAGGAAT-3' was added into 4.5pmol of single-stranded DNA of IB libraries. The volume of the reaction system was 150ul, containing 30mM Tris-HCl (pH7.5), 50mM NaCl, 15mM MgCl₂, 1mM DTT, 0.1mM dNTPs, 2.5mM ddATP, ddCTP, ddGTP and trace of [α³²P]dCTP. The mixture was placed in water at 60 °C for 5 minutes, then 50 °C for 15 minutes, then cooled down to 37 °C ; after adding 75 units of Klenow DNA polymerase (United states Biochemical), placed again in water for 30 minutes. EDTA was added to stop the reaction. The mixture was extracted with phenol/chloroform, then diluted with 2ml HAP loading buffer containing 50ug ultrasonicated denatured salmon sperm DNA carrier and passed through hydroxyapatite chromatography column (as mentioned above). Some of the double-stranded DNA bound to the hydroxyapatite was eluted with 6ml eluting solution (0.4M sodium phosphate buffer, pH6.8/10mM EDTA/1%SDS). Added 14ml water containing 50ug DNA carrier, regulated the concentration of the phosphoric acid to 0.12M, and repeated the above steps. Finally, using the method mentioned above, the obtained eluent was extracted and desalinated, then the DNA was precipitated with ethanol. The precipitate (112ng) was dissolved in 2.5ul formamide, added a drop of mineral oil and heated at 80 °C for 3 minutes to denature DNA. 0.5ul Tris-HCl (pH7.5), 0.1MEDTA, 0.5ul 5M NaCl, lul (5ug) (dT)₂₅₋₃₀, and 0.5ul (0.5ug) extending primer were added and the whole volume of the reaction system was adjusted to 5ul. Subsequently, the anneal reaction was carried on. Finally the two components were used to block the adenine residue (i.e., the original poly(A) tail) and extend the oligonucleotide chain on the single-stranded annular DNA.

The annealed mixture was incubated at 42 °C , after the below mentioned time intervals, 2.5ul sample was taken out each time:
1 hour later, 2.5ul sample was taken out (calculated C₀t=0.42); (A₁⁺⁺⁺)
2.36 hours later, 2.5ul sample was taken out (calculated C₀t=1). (A₂⁺⁺⁺)

The 2.5ul samples were treated by hydroxyapatite chromatography so as to separate the unbound single-stranded annular DNA from some of the re-bound double-stranded DNA, which were transformed into two highly repeated cDNA homogenized sublibraries (A₁⁺⁺⁺ and A₂⁺⁺⁺), respectively. The single-stranded annular DNA was obtained from the elution peak (as mentioned above). The concentration of the obtained single-stranded annular DNA was measured with spectrophotometer and the above steps were repeated.

The annealed mixture was incubated at 42 °C , after the below mentioned time intervals, 1.5ul sample was taken out each time:
13 hour later, 1.5ul sample was taken out (calculated C₀t=5.5); (A₁⁺⁺)
23.6 hours later, 1.5ul sample was taken out (calculated C₀t=10); (A₂⁺⁺)
70.8 hours later, 1.5ul sample was taken out (calculated C₀t=30); (A₃⁺⁺)

The 2.5ul samples were treated by hydroxyapatite chromatography so as to separate the unbound single-stranded annular DNA from the partial re-bound double-stranded DNA, which were transformed into three moderately repeated cDNA homogenized sublibraries (A₁^{++.} A₂⁺⁺ and A₃⁺⁺), respectively. The single-stranded annular DNA was obtained from the elution peak (as mentioned above). The concentration of the obtained single-stranded annular DNA was measured with spectrophotometer; and the above steps were repeated.

The annealed mixture was incubated at 42 °C , after the below mentioned time intervals, 2.5ul sample was taken out each time:
118 hour later, 2.5ul sample was taken out (calculated C₀t=50); (A₁⁺)
188 hours later, 2.5ul sample was taken out (calculated C₀t=80); (A₂⁺)

The two 2.5ul samples were treated by hydroxyapatite chromatography so as to separate the unbound single-stranded annular DNA from some of the re-bound double-stranded DNA, which were transformed into two low repeated cDNA homogenized sublibraries (A₁⁺ and A₂⁺), respectively. The single-stranded annular DNA was obtained from the elution peak (as mentioned above) and converted into two rare cDNA homogenized sublibraries (A₁⁻ and A₂⁻). Because the electoporation efficiency of partial double-stranded DNA was 100 times higher than that of the single-stranded DNA (Rubenstein, J.L.R., Brice, A.E.J., Ciaranello, R.D., Denney, D., Porteus, M.H. & Usdin, T.B. (1990) Nucleic Acid Res. 18, 4833-4842.), the random hexamer and T₇DNA polymerase (Sequenase version II; United States Biochemical) were added to extend the primer, and the single-stranded annular DNA was converted into partial double-stranded DNA. The volume of the reaction mixture was 10-20ul, containing 1mM dNTPs. EDTA was added to stop the reaction (final concentration was 20mM). After having been extracted with phenol and precipitated with ethanol, the obtained cDNA was dissolved into 10mM Tris-HCl, pH7.5/1mM EDTA, then transformed into competence host (DH10, GIBCO/BRL) by electroporation. In order to measure the number of the transformants, 1 hour after electroporation, 10ul of bacteria was coated onto a LB plate containing 75ug/ml ampicillin. The measurement showed that a homogenized library containing 2.5 x 10⁶ clones was obtained. After extraction with Qiagen plasmid kit(Qiagen, Chatsworth, CA), super helix plasmid DNA could be obtained.

### Construction of homogenized and subtracted cDNA sublibraries only expressed in healthy infant brain

The homogenizing steps were the same as those mentioned above, except the subtracted homogenizing treatment with synthesized fragments which used cDNA original libraries as template and the constructed human infant brain A₁⁺⁺⁺ and A₂⁺⁺⁺, the cDNA original libraries were: human embryonic liver and spleen, human full-term placenta, human 8-9 weeks' placenta, human breast, adult brain, human retina, human conarium, human oophoroma, human melanocyte, human embryonic heart, human parathyroid cancer, human senile fibroblast, human poly-sclerotic focus, human embryonic lung.

The annealed mixture was incubated at 42 °C. Then, the samples whose Cₒt were 0.42 and 1 respectively were taken to construct two homogenized sublibraries. Thus, the cDNA enriching on the HAP column was the cDNA highly expressed in both of cDNA original libraries, while the elution peak was the abundant cDNA only expressed in A⁺⁺⁺(A represented human infant brain). These two homogenized sublibraries were designated as: spA₁⁺⁺⁺ and spA₂⁺⁺⁺.

The A⁺⁺ libraries were subtracted and homogenized in the same way. The annealed mixture was incubated at 42 °C , while taking the samples whose Cₒt were 5.5, 10 and 30, respectively. By collecting the elution peak, three moderate abundance homogenized sublibraries only expressed in A (A represented human infant brain) were obtained, and designated as spA₁⁺⁺,sp A₂⁺⁺ and spA₃⁺⁺.

The A⁺ and A⁻ were subtracted and homogenized in the same way. The annealed mixture was incubated at 42 °C, while taking the samples whose Cₒt were 50 and 80, respectively. By collecting the elution peak, two low abundance homogenized libraries only expressed in A (A represented human infant brain) were obtained, and designated as spA₁⁺ and spA₂⁺. Further two very low abundance homogenized libraries were obtained, and designated as: spA₁⁻ and spA₂⁻.

For the sublibraries obtained from each step, the large-scale sequencing were carried out by judging whether the cDNA clones were novel; If yes, analyzing the integrity of 5'-terminal until full-length sequence was obtained. All the sequenced long cDNA clones formed the existing cDNA libraries. Then the probe were synthesized, hybridized with the obtained sublibraries and original libraries to eliminate the sequenced cDNA clones and retain unsequenced clones so as to improve the efficiency of the large-scale sequencing. The simple process was shown in Figure 3. The method of hybridization was as follows.

### Colony hybridization

The duplicate nylon filters (GeneScreenPlus; DuPond/NEN) was treated according to the method of Grunstein, M. & Hogness, D. (1975) Proc. Acad. Sci. USA 72, 3961-3965. The colonies were allow to grow on the membrane so as to screen the libraries. Hybridization was carried on at 42 °C . The reaction system contained 50% formamide, 5 x Denhardt's solution, 0.75M NaCl, 0.15M Tris-HCl, pH7.5, 0.1M sodium phosphate, 2% SDS, and 100ug/ml digested and degenerated salmon sperm DNA. Radioactive probes were prepared with synthetic primers and Prime-it II kit (Stratagene)(Feinberg, A.P.& Vogelstein, B. (1983) Anal. Biochem. 132, 6-13. Feinberg, A.P.& Vogelstein, B. (1984) Anal. Biochem. 137, 266-267).

### DNA sequencing

The double-stranded plasmid DNA template was prepared by Wizard Minipreps DNA purification system (Promega), sequenced from both terminals with forward and backward M13 fluorescent primers. The reaction products were measured by Biomek 1000 workstation (Beckman). Then the products were transferred to PCR amplifier (Perkin-Elmer/Cetus) to conduct cycling measurement. The reaction products were analyzed by 370 DNA automatic sequencer (Applied Bios). The research of nucleic acid and protein databases was carried out using BLAST algorithm (Altschul, S.F., W. Niller, E. Myers, and D.J. Lipman. 1990. Basic local alignment search tool. J.Mol.Biol. 215: 403-410.) on the server of the National Center for Biotechnology Information.

### Example 2:

### Construction of homogenized and subtracted cDNA sublibraries only or mainly expressed in infant (72 days) brains of spinomyotrophic syndrome and the large scale sequencing

### Construction of high quality cDNA original libraries from different tissues or cells

According to the manufacturer's instruction, Oligotex mRNA kit was used to purify poly(A)+ RNA from infant (72 days) brains of spinomyotrophic syndrome and other cells, except the senile fibroblast used to separate cytoplast RNA. Twice purification was needed. The construction of cDNA libraries was very important. In typical reactions, 1ug of poly(A)+RNA and 2 times of oligo(dT)₁₈-Not I primer (PacI primer was used for liver/spleen libraries) were annealed at 37 °C and then reverse transcribed at 37 °C with reverse transcriptase (Life Technologies). Poly(A)+RNA also could be annealed by adding 4 times of (dT)₂₅-Not I primer at 45 °C and then reverse transcribed at 45 °C . Tag is a sequence of 2-6 nucleotides which was specific for the different libraries. So it could be used to identify libraries. Except infant brain, embryonic liver/spleen, and placenta libraries, the first strand of cDNA of all the other libraries was synthesized using the following oligonucleotide primer: TGTTACCAATCTGAAG-TGGGAGCGGCCGC-tag-(dT)₁₈or₂₅. First strand of cDNA of embryonic heart, brain and placenta libraries was synthesized using the following primer: AACTGGAAGAATTAATTAAAG-ATCT(dT)₁₈ (Pharmacia). AACTGGAAGAAT-TAATTAAAGATCT(dT)₁₈ was used as the primer in the synthesis of the first strand of cDNA of embryonic liver/spleen. Double-stranded cDNA was filtrated and separated by polyacrylamide gel column (length 64 cm, diameter 0.2cm) and then ligated with 500 to 1000 times linkers. Infant brain cDNA was ligated by Hind III linker and cut by Not I, separated by polyacrylamide gel column again and finally cloned to the site between NotI and Hind III of vector Lafmid BA ( Soares, M.B.1994. Construction of directionally cloned cDNA libraries in phagemid vectors. In Automated DNA sequencing and analysis (ed. M.D.Adams, C.Fields and J.C.Venter), pp.110-114. Academic Press, New York,NY.USA 74,1020-1023.). Embryonic liver/spleen cDNA libraries were ligated by EcoRI linker and cut by PacI and then cloned between PacI and EcoRI of vector pT7T3-Pac (Pharmacia). pT₇T₃-Pac was a modified polylinker like pT7T3-Pac18D and could transform E.coli and construct cDNA original libraries from different tissues or cells. Plasmid DNA from the cDNA original libraries was purified on HPLC reverse-phase column and affinity column, transformed via electroporation into DH5αF'. The following method was used get different single-stranded and double-stranded high quality cDNA original libraries.

The following was the sequence of pT7T3-Pac polylinker:

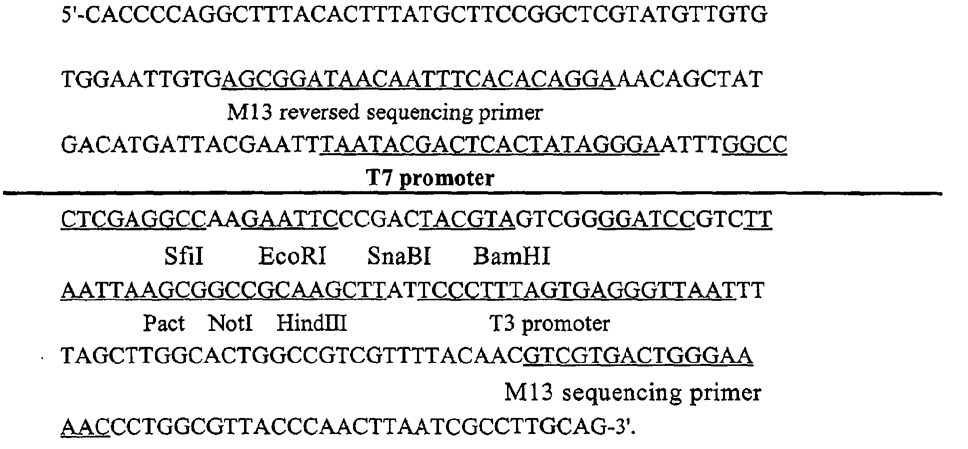

### Construction of purified and high quality single-stranded annular DNA original libraries in vitro

According to manufacturer's instruction(Life Technologies,Catalogue10356-020), use Gene II (phagemid F1 endonuclease) and E.coli exonuclease III (Exo III) to convert phage's double-stranded DNA into single-stranded DNA. As described above ( Soares, M.B.1994.Construction of directionally cloned cDNA libraries in phagemid vectors. In Automated DNA sequencing and analysis (ed. M.D.Adams, C.Fields and J.C.Venter),pp.110-114. Academic Press, New York,NY.USA 74,1020-1023.),the derived single-stranded DNA can be purified by HAP chromatography (Bio-Rad) from residual double-stranded plasmid DNA. The original protein of phage F1 replication is a kind of specific endonuclease that can bind to phage F1 start site and make a cut in the superhelix DNA. The strand having the cut can be cleavaged later by Exo III and form single-stranded loop (Hoheisel,J.D. 1993. On the activities of Escherichia coil exonuclease III. Anal.Biochem. 209: 238-246.). Gene II's function of loosing superhelix plasmid is not completed so HAP is needed to purify single-stranded loop. GeneII reacted for 1h at 37 °C and a typical reaction includes 4ul superhelix plasmid of cDNA libraries, lul GeneII (Life Technologies), 2ul 10 x GeneII buffer (Life Technologies), totally 20ul. GeneII was deactivated at 65 °C for 5 min, cooled on ice and then 2ul Exo III was added, incubated for 30 min at 37 °C , GeneII and Exo III (Life Technologies, Catalogue18013-011,65u/ul) were cut by protease K (Boehringer Mannheim) at 50 °C for 15 min. The total volume was 100ul, including 10mM Tris (PH7.8), 5mM EDTA, 0.5% SDS and 136ul protease K. Then it was eluted with phenol, chloroform and isoamyl alcohol (25: 24: 1) of the same volume, precipitated with ethanol, reacted with PvuII at 37 °C for 2h and converted the residual superhelix plasmid into linearized DNA to increase the affinity with HAP. PuvII could not cut single strand, so there were two PuvII sites in vector. The reacted mixture was diluted by adding 2ul buffer [0.12M sodium phosphate (PH6.8), 10mM EDTA and 1% SDS] and purified with HAP affinity chromatography pre-equilibrated by the same buffer (bed volume is 1ml or 0.4g HAP) at 60 °C. The mixture was washed with 6ml buffer and combined the washed buffer with the flow-though, extracted with saturated 2-butanol 2 times, anhydrous 2-nbutanol once and saturated ether once (Three times of the volume were used each time). Residual ether was dried by vacuum, and desalinated on a Nensorb column (DuPont/NEN) according to manufacturer's instruction and finally concentrated to 0.35 ml and precipitated with ethanol. It should be pointed out that the polarity of single-stranded DNA made by GeneII and Exo III is opposite to that of the single-stranded DNA produced in the phagemid.

### Production of high quality covalently closed single-stranded cDNA original libraries in vivo

According to the method described in the reference (Vieira, J.& Messing, J.(1978) Methods Enzymol.153,3-11.), plasmid DNA from original libraries was transformed into E.coli DH5αF' by electroporation, and cultured to OD₆₀₀ under the selection of ampicillin at 37 °C and super-infected by 10-20 times of helper phage M13KO₇ (Pharmacia). Single-stranded annular plasmid was harvested after 4 hours of cultivation.

### Converting the single-stranded loop into the double-stranded plasmid

Single-stranded loop was precipitated by ethanol (<50ug) and resuspended in 11ul water. After adding 4ul 5 x sequencing enzyme buffer (USB) and 1ul primer (1ug), the mixture was incubated at 65 °C for 5 min. at 37 °C for 3 min. The mixture was then incubated at 37 °C for 30 min before adding 1ul sequencing enzyme (UBS 2.0),1ul 0.1M DTT, 2ul mixed dNTP ( The final concentration of each dNTP was 10mM). The mixture was extracted by phenol, chloroform and isoamyl alcohol (25: 24: 1). Added 10mM Tris(PH8.0) and 1mM EDTA(TE) until the total volume was 100ml.

Plasmid DNA was precipitated by ethanol and dissolved into 3ul TE. Oligonucleotides as follows were used in the reaction of primer elongation:
(1) M13 reversed sequencing primer (5'-AGCGGATAACAATTTCACACAGGA-3'), which was complemented to the single strand outside the bodies.
(2) Oligomeric Amp(5'-GACTGGTGAGTACTCAA-CCAAGTC-3'), which was complementary to single-stranded pT₇T₃-Pac from bodies or single-stranded Lafmid BA plasmid of anti-ampicillin gene.

These high quality cDNA libraries established as described had the following characteristics: the average length of inserted fragments was 1.7kb; the genetic information of coding region usually could be obtained by sequencing the 5' end; the poly(A) tail of mRNA was short; more useful information could be obtained by sequencing the 3' end; the occurrence of non-recombined clone was very few (0.01%); sequencing more than 2000 clones shown no chimeric cDNA.

### Construction of cDNA homogenized sublibraries

This is a method based on the renaturation dynamics. This method is to hybridize excessive 20 times inserted cDNA fragments produced from PCR with single-stranded libraries, purify the residual single-stranded plasmid with HAP and convert them into double strand, transform them into DH10B by electroporation and finally select them by ampicillin. According to the instruction, PCR amplification of inserted cDNA fragment was carried out in an amplification system of high fidelity (Boehringer Mannheim) which contains a mixture of Taq and Pwo DNA enzymes (Barnes, W.M.1994. PCR amplification of up to 35-kb DNA with high fidelity and high yield from lambda bacteriophage templates. Proc.Natl.Acad.Sci. 91: 2216-2220.). 1ul (2.5-5.0) DNA template [double-stranded plasmid (cDNA original libraries of infant brains of spinomyotrophic syndrome, embryonic lung, hypoparathyroid adenoma, senile fibroblast) or single-stranded annular DNA (cDNA original libraries of embryonic heart, embryonic liver/spleen) prepared ex vivo] and 2ul reserved dNTP solution (final concentration of each dNTP was 200Um in reaction), 5ul 20uM T₇ primer (5'-TAATACGACTCACTATAGGG-3'), 5ul 20uM T₃ primer (5'-ATTAACCCTCACTAAAGGGA-3'), 10ul 10 x buffer of the high fidelity amplification system, 0.75ul enzyme(2.6 u) of the high fidelity amplification system, 76.25ul water and 50ul mineral oil were added. The reaction was carried out in a Perkin Elmer cycler. The reaction was performed under the following conditions: first rise from room temperature to 94 °C for 7 min. incubate at 94 °C for 1 min, at 55° C for 2 min, at 72 °C for 3 min, at 72 °C for 7 min. and 20 cycles. Fragments produced by PCR were purified by high purity PCR product purification kit, and then dissolved into 5ul TE. 1.5ul(0.5ug) was taken and mixed with 5ul(50ug) DNA (single-stranded annular DNA produced in vitro), deionized formamide, 0.5ul(10ug) 5' blocking oligomer AV-1 (5'-CCTCGTGCCGAATTCTTGGCCTCGAGG GCCAAATTCCCTATAGTGAGTGTATTA-3'), 0.5ul (10ug) 3' blocking oligomer AR (5'-ATTAACCCT-CACTAAA-GGGAATAAGCTTGCGGCCGCT₂₀-3', which was used for all the libraries except embryonic liver/spleen) or 0.5ul(10ug)3' AV-2(5'-ATTAACCCT CACTAAAGGGAATAAGCTTGCGGCCGCTTAATTAAAGATCT₁₉-3' only used for embryonic liver/spleen), and sealed with 10ul mineral oil. The mixture was heated at 80 °C for 3 min. Added lul 10 x buffer-A [1.2M NaCl, 0.1M Tris(PH8.0) and 50mM EDTA, for cDNA original libraries of infant brains of spinomyotrophic syndrome, embryonic lung, embryonic heart, hypoparathyroid adenoma, senile fibroblast] or add 1ul buffer-B [1.2M NaCl, 0.1M Tris(PH8.0), 50mM EDTA and 10% SDS, used for cDNA original libraries of 14Nb2HFL20W- embryonic liver/spleen] and 1.5ul water. Hybridizing reaction was performed at 30 °C and the following time or calculated C₀t. Residual single-stranded DNA was purified by HAP, converted into double strand, and transformed into DH10B by electroporation.

Following example is used to explain the construction of the homogenized cDNA sublibraries and B denotes cDNA original libraries of infant brains of spinomyotrophic syndrome. The annealed mixture described above was incubated at 30 °C and 2.5ul of samples were taken at following time intervals:
Take 2.5ul sample after 0.17 hour(calculated C₀t=0.42), (B₁⁺⁺⁺)
Take 2.5ul sample after 0.4 hour(calculated C₀t=1), (B₂⁺⁺⁺)

The 2.5ul samples were treated by hydroxyapatite chromatography so as to separate the non-hybridized single-stranded annular DNA from some of the re-bound double-stranded DNA, which were transformed into two highly repeated cDNA homogenized sublibraries (B₁⁺⁺⁺ and B₂⁺⁺⁺), respectively. The single-stranded annular DNA was obtained from the elution peak (as mentioned above). The concentration of the obtained single-stranded annular DNA was measured with spectrophotometer and the above steps were repeated.

The annealed mixture was incubated at 42 °C and 1.5ul of sample was taken at following time intervals:
Take 1.5ul sample after 2.2 h.(calculated C₀t=5.5), (B₁⁺⁺)
Take 1.5ul sample after 4 h.(calculated C₀t=10), (B₂⁺⁺)
Take 1.5ul sample after 12 h.(calculated C₀t=30),(B₃⁺⁺)

The 1.5ul samples were treated by hydroxyapatite chromatography so as to separate the non-hybridized single-stranded annular DNA from some of the re-bound double-stranded DNA, which were transformed into three moderately repeated cDNA homogenized sublibraries (B₁⁺⁺, B₂⁺⁺ and B₃⁺⁺), respectively. The single-stranded annular DNA was obtained from the elution peak (as mentioned above). The concentration of the obtained single-stranded annular DNA was measured with spectrophotometer and the above steps were repeated.

The annealed mixture was incubated at 42 °C and 2.5ul of sample was taken at following time intervals:
Take 2.5ul sample after 20.h. (calculated C₀t=50), (B₁⁺)
Take 2.5ul sample after 32.h. (calculated C₀t=80), (B₂⁺)

The two 2.5ul samples were treated by hydroxyapatite chromatography so as to separate the non-hybridized single-stranded annular DNA from some of the re-bound double-stranded DNA, which were transformed into two low repeated cDNA homogenized sublibraries (B₁⁺ and B₂⁺), respectively. The single-stranded annular DNA was obtained from the elution peak (as mentioned above) and converted into two rare cDNA homogenized sublibraries (B₁⁻ and B₂⁻). Because the electoporation efficiency of partial double-stranded DNA was 100 times higher than that of the single-stranded DNA (Rubenstein, J.L.R., Brice, A.E.J., Ciaranello, R.D., Denney, D., Porteus, M.H. & Usdin, T.B. (1990) Nucleic Acid Res. 18, 4833-4842.), the random hexamer and T₇DNA polymerase (Sequenase version II; United States Biochemical) were added to extend the primer, and the single-stranded annular DNA was converted into partial double-stranded DNA. The volume of the reaction mixture was 10-20ul, containing 1mM dNTPs. EDTA was added to stop the reaction (final concentration was 20mM). After having been extracted with phenol and precipitated with ethanol, the obtained cDNA was dissolved into 10mM Tris-HCl, pH7.5/1mM EDTA, then transformed into competence host (DH10, GIBCO/BRL) by electroporation. In order to measure the number of the transformants, 1 hour after electroporation, 10ul of bacteria was coated onto a LB plate containing 75ug/ml ampicillin. The measurement showed that a homogenized library containing 2.5 x 10⁶ clones was obtained. After extraction with Qiagen plasmid kit(Qiagen, Chatsworth, CA), super helix plasmid DNA could be obtained.

### Constitution of homogenized cDNA libraries expressed only or mainly in infant brain cells of spinal atrophia

The following is the subtraction of the constituted homogenized cDNA libraries for infant brain cells of spinal atrophia. According to the description, double-stranded cDNA plasmid may be prepared using Olagen Midi-prep kit. These DNAs come from high-class cDNA original libraries of human normal infant brain, adult brain, human fetal liver and spleen, human full-term placenta, human 8-9 weeks placenta, human breast, human retina, human pineal gland, human oophoroma, human melanocyte, human fetal heart, human parathyroid cancer, human senility fibroblast, human multiple scleroderma focus, human fetal lung, etc. Then double-stranded plasmid DNA is converted into single strand in vitro. As mentioned above, single-stranded cyclic DNA was purified by HAP chromatography and used as the template of PCR amplification with primer T7 and T3. The single-stranded cyclic DNA from constituted homogenized cDNA libraries (in 2ul deionized formamide) for infant brain cells of spinal atrophia was blended with the amplified DNA inserted fragments obtained from the whole 1.5ug cDNA original libraries (in 4ul deionized formamide). After adding 2.1 ul (42 ug) of 5' blocking oligomer AV-1 and 2.1 ul (42ug) 3' blocking oligomer AV-2, 10ul mineral oil was added. Heated 30 minute at 80 °C, and then added 1.2 ul of 10 x buffer B and 0.6 ul of water. The hybridization was carried out at 30 °C and the following time or calculated C₀t. The non-hybridized single-stranded DNA was purified by HAP, converted into double strand, and transformed into DH10B by electroporation. After selection with ampicillin, the subtracted and homogenized cDNA libraries only or mainly expressed in infant brain cells of spinal atrophia were obtained. The DNAs bound with HAP were treated and purified in the same way in order to control the experiment.

The above DNAs were annealing with B1⁺⁺⁺and B2⁺⁺⁺ at 30 °C for 0.73 hour and 1.8 hours, respectively. The corresponding C₀t values were 0.42 and 1, respectively. In this way cDNAs enriched in HAP were those highly expressed in all cDNA original libraries. Those in elution peaks were cDNAs that were only highly expressed in B⁺⁺⁺. These libraries were designated as spB1⁺⁺⁺ and spB2⁺⁺⁺.

The B⁺⁺ libraries were subtracted and homogenized in the same way. The annealed mixture was incubated at 30°C for 9.7h, 17.7h and 53.3h, which corresponded to Cₒt values of 5.5, 10 and 30, respectively. By collecting the elution peak, three moderate abundance homogenized sublibraries only expressed in B were obtained, and designated as spB₁⁺⁺,sp B₂⁺⁺ and spB₃⁺⁺.

The B⁺ and B⁻ were subtracted and homogenized in the same way. The annealed mixture was incubated at 30 °C for 88.9h and 142h, which corresponded to Cₒt values of 50 and 80, respectively. By collecting the elution peak, two low abundance homogenized libraries only expressed in B were obtained, and designated as spB₁⁺ and spB₂⁺. Further two very low abundance homogenized libraries were obtained and designated as: spB₁⁻ and spB₂⁻.

For the sublibraries obtained from each step, the large-scale sequencing were carried out by judging whether the cDNA clones were novel; If yes, analyzing the integrity of 5'-terminal until full-length sequence was obtained. All the sequenced long cDNA clones formed the existing cDNA libraries. Then the probe were synthesized, hybridized with the obtained sublibraries and original libraries to eliminate the sequenced cDNA clones and retain unsequenced clones so as to improve the efficiency of the large-scale sequencing. The simple process was shown in Figure 3. The method of hybridization was as follows.

### Colony hybridization

The duplicate nylon filters (GeneScreenPlus; DuPond/NEN) was treated according to the method of Grunstein, M. & Hogness, D. (1975) Proc. Acad. Sci. USA 72, 3961-3965. The colonies were allow to grow on the membrane so as to screen the libraries. Hybridization was carried on at 42°C. The reaction system contained 50% formamide, 5 x Denhardt's solution, 0.75M NaCl, 0.15M Tris-HCl, pH7.5, 0.1M sodium phosphate, 2% SDS, and 100ug/ml digested and degenerated salmon sperm DNA. Radioactive probes were prepared with synthetic primers and Prime-it II kit (Stratagene)(Feinberg, A.P.& Vogelstein, B. (1983) Anal. Biochem. 132, 6-13. Feinberg, A.P.& Vogelstein, B. (1984) Anal. Biochem. 137, 266-267).

### DNA sequencing

The double-stranded plasmid DNA template was prepared by Wizard Minipreps DNA purification system (Promega), sequenced from both terminals with forward and backward M13 fluorescent primers. The reaction products were measured by Biomek 1000 workstation (Beckman) Then the products were transferred to PCR amplifier (Perkin-Elmer/Cetus) to conduct cycling measurement. The reaction products were analyzed by 370 DNA automatic sequencer (Applied Bios). The research of nucleic acid and protein databases was carried out using BLAST algorithm (Altschul, S.F., W. Niller, E. Myers, and D.J. Lipman. 1990. Basic local alignment search tool. J.Mol.Biol. 215: 403-410.) on the server of the National Center for Biotechnology Information.

The efficiency of obtaining new sequences was well over 10% through 50 cycles of sequencing and subtractive hybridization.

All the documents cited herein are incorporated into the invention as reference, as if each of them is individually incorporated. Further, it is appreciated that, in the above teaching of the invention, the skilled in the art can make certain changes or modifications to the invention, and these equivalents are still within the scope of the invention defined by the appended claims of the present application.

## Claims

1. A method of cDNA sequencing which comprises the steps of:
(1) constructing cDNA original libraries in which the length of the inserted fragments is 0.5-3.0 kb, by using tissues of organisms as materials;
(2) homogenizing the cDNA original libraries of step (1) according to the graded C0t value, thereby obtaining the homogenized cDNA libraries corresponding to the graded C₀t, wherein C₀ is concentration of the total DNA and the unit thereof is mol/L based on the number of nucleic acids; and t is the renaturing time and the unit thereof is second;
(3) selecting and sequencing 5-500 clones from each of the homogenized cDNA libraries of the preceding step;
(4) synthesizing probes corresponding to the sequenced sequences by using the sequenced cDNA clones, and hybridizing and subtracting the homogenized cDNA libraries of the preceding step with said probes, thereby removing the sequenced cDNA clones from the cDNA libraries and forming the subtracted homogenized cDNA libraries;
(5) repeating Steps (3) and (4) 1-5,000 times.

2. The method of claim 1, wherein in steps (1) and (2),
using the different tissues of the organism as materials to construct cDNA original libraries of different tissues;
homogenizing said cDNA original libraries respectively to obtain homogenized libraries of different tissues;
hybridizing and subtracting among said homogenized cDNA libraries of different tissues, thereby forming the homogenized cDNA libraries subtracted by hybridization.

3. The method of claim 2, wherein said different tissues comprises: tissues of different types in the same organism, tissues of different developing phases in the same organism, normal tissues and pathological tissues in the same organism, and tissues in different organisms.

4. The method of claim 1, wherein Step (4) further comprises:
synthesizing probes based on the known cDNA sequences, or synthesizing probes based on other sequenced clones; and hybridizing and subtracting the homogenized cDNA libraries of the preceding step with said probes.

5. The method of claim 1, wherein in Step (3), DNA sequencing comprises the following steps:
for the sequenced fragments, determining whether they are new cDNA clones;
for the new clones, analyzing the integrity of 5' end;
for the new clones having the integral 5' end, sequencing until obtaining the full-length sequences.

6. The method of claim 1, wherein in Step (2), C₀t is divided into 3-8 grades.

7. The method of claim 6, wherein C₀t is divided into 3 grades: 0 < C₀t ≼ 1, C₀t =1-50, and C₀t ≽ 50, thereby obtaining the homogenized libraries selected from the group consisting of (a) highly repeated homogenized cDNA libraries, (b) moderate repeated homogenized cDNA libraries, and (c) lowly repeated homogenized cDNA libraries.

8. The method of claim 1, wherein in step (1), constructing cDNA original libraries comprises the following steps:
extracting mRNA, amplifying mRNA to obtain the corresponding cDNA by a technique selected from the group consisting of: mixed reverse transcriptase technique, SMART PCR technique, nucleotide capping technique and the combination thereof;
separating and collecting cDNA fragments of 0.5-3.0kb;
cloning the separated cDNA fragments into proper vectors;
separating the vectors comprising the inserted fragments of 0.5-3.0kb;
transforming into proper bacteria, thereby obtaining the cDNA original libraries.

9. The method of claim 1, wherein in step (1), constructing cDNA original libraries comprises the following steps:
extracting mRNA, amplifying mRNA to obtain the corresponding cDNA by a technique selected from the group consisting of: mixed reverse transcriptase technique, SMART PCR technique, nucleotide capping technique and the combination thereof;
separating and collecting cDNA fragments of 0.5-3.0kb;
cloning the separated cDNA fragments into proper vectors;
separating the vectors comprising the inserted fragments of 0.5-3.0kb;
transforming into proper bacteria, thereby obtaining the cDNA libraries.
extracting recombinant DNA from the cDNA libraries, passing through a poly(T)₁₀₋₂₅ affinity chromatographic column, collecting the recombinant cDNA bound with poly(T)₁₀₋₂₅ and transferring said cDNA into proper bacteria, thereby obtaining the cDNA original library.

10. The method of claims 8 or 9, wherein the separation and collection of cDNA fragments of 0.5-3.0kb are carried out by electrophoresis and cutting gel, or by gel chromatography purification; and the separation of the vectors comprising the inserted fragments of 0.5-3.0kb is carried out by reversed phase HPLC.
